(19) Europäisches Patentamt
European Patent Office
Office européen des brevets

(11) Veröffentlichungsnummer : **0 348 734 B1**

(12) **EUROPÄISCHE PATENTSCHRIFT**

(45) Veröffentlichungstag der Patentschrift : **21.10.92 Patentblatt 92/43**

(51) Int. Cl.$^5$ : **A01N 43/82,** C07D 285/12, C07D 417/12

(21) Anmeldenummer : **89110796.3**

(22) Anmeldetag : **14.06.89**

(54) **Neue Difluormethyl-thiadiazolyl-oxyessigsäureamiden.**

Die Akte enthält technische Angaben, die nach dem Eingang der Anmeldung eingereicht wurden und die nicht in dieser Patentschrift enthalten sind.

(30) Priorität : **27.06.88 DE 3821597**

(43) Veröffentlichungstag der Anmeldung : **03.01.90 Patentblatt 90/01**

(45) Bekanntmachung des Hinweises auf die Patenterteilung : **21.10.92 Patentblatt 92/43**

(84) Benannte Vertragsstaaten : **BE CH DE ES FR GB IT LI NL SE**

(56) Entgegenhaltungen :
**EP-A- 0 148 501**
**EP-A- 0 298 338**
**US-A- 4 005 213**

(73) Patentinhaber : **BAYER AG**
**W-5090 Leverkusen 1 Bayerwerk (DE)**

(72) Erfinder : **Förster, Heinz, Dr.**
**Am Eckbusch 47**
**W-5600 Wuppertal 1 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch Gladbach 2 (DE)**
Erfinder : **Strang, Harry, Dr.**
**Unterdorfstrasse 6A**
**W-4000 Düsseldorf 31 (DE)**

EP 0 348 734 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-(5-Difluormethyl-1,3,4-thiadiazol-2-yl-oxy)-essigsäure-N-isopropyl-anilide (nachfolgend vereinfacht als "Difluormethyl-thiadia=zolyl-oxyessigsäureamide" bezeichnet), ein Verfahren zu ihrer Herstellung und ihre Verwendung als selektive Herbizide für verschiedene Nutzpflanzenkulturen.

Es ist bereits bekannt, daß bestimmte Heteroaryloxyessigsäureamide, wie z, B, 2-(Benzthiazol-2-yl-oxy)-essigsäure-N-methylanilid, herbizide Eigenschaften aufweisen (vgl, EP-A 5501 und US-P 4 509 971). Die herbizide Wirksamkeit der vorbekannten Verbindungen ist jedoch nicht immer ganz zufriedenstellend.

Weiter sind bestimmte Difluormethyl-thiadiazolyl-oxyessigsäureamide beschrieben worden (vgl, EP-A-148 501 und US-P 4 708 731). Über die konkreten herbiziden Eigenschaften dieser Verbindungen, insbesondere über eventuell vorhandene selektiv-herbizide Eigenschaften ist bisher jedoch nichts bekanntgeworden.

Es wurde nun gefunden, daß die neuen Difluormethyl-thiadiazolyl-oxyessigsäureamide der allgemeinen Formel (I)

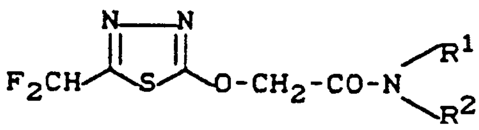

(I)

in welcher

$R^1$ für Isopropyl steht und

$R^2$ für gegebenenfalls durch Fluor, Chlor, Brom, Methyl, Ethyl, Trifluormethyl, Methoxy und/oder Ethoxy substituiertes Phenyl steht,

starke herbizide Wirkung bei guter Nutzpflanzenselektivität aufweisen.

Überraschenderweise zeigen die neuen Difluormethyl-thiadiazolyl-oxyessigsäureamide der allgemeinen Formel (I) erheblich stärkere herbizide Wirkung gegen verbreitete, schwer bekämpfbare Unkräuter als die oben genannten Verbindungen bei guter Verträglichkeit gegenüber wichtigen Kulturpflanzen.

Beispiele für die erfindungsgemäßen Verbindungen der Formel (I) sind in der nachstehenden Tabelle 1 aufgeführt.

Tabelle 1: Beispiele für die Verbindungen der Formel (I)

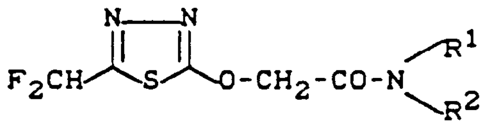 (I)

| Beisp.-Nr. | $R^1$ | $R^2$ bzw. 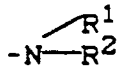 | Physikalische Daten |
|---|---|---|---|
| 1 | $CH(CH_3)_2$ | 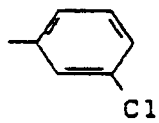 | Fp.: 98 °C |
| 2 | $CH(CH_3)_2$ | 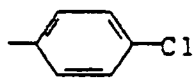 | Fp.: 69 °C |
| 3 | $CH(CH_3)_2$ | 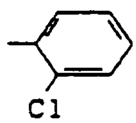 | Fp.: 113 °C |
| 4 | $CH(CH_3)_2$ | 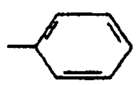 | Fp.: 123 °C |

Tabelle 1 - Fortsetzung

| Beisp.-Nr. | $R^1$ bzw. $-NR^1R^2$ | $R^2$ | Physikalische Daten |
|---|---|---|---|
| 5 | $CH(CH_3)_2$ | 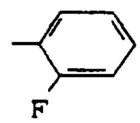 | Fp.: 128° C |
| 6 | $CH(CH_3)_2$ | 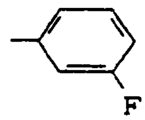 | Fp.: 120° C |
| 7 | $CH(CH_3)_2$ | 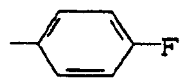 | Fp.: 97° C |
| 8 | $CH(CH_3)_2$ | 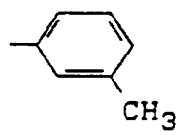 | Fp.: 95° C |
| 9 | $CH(CH_3)_2$ | 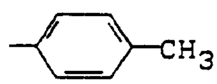 | |

Tabelle 1 - Fortsetzung

| Beisp.-Nr. | $R^1$ bzw. $-NR^1R^2$ | $R^2$ | Physikalische Daten |
|---|---|---|---|
| 10 | $CH(CH_3)_2$ | 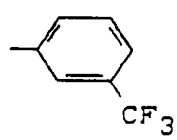 | |
| 11 | $CH(CH_3)_2$ | 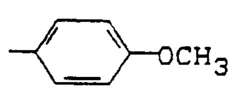 | Fp.: 66° C |

Tabelle 1 - Fortsetzung

| Beisp.-Nr. | $R^1$ | $R^2$ | Physikalische Daten |
|---|---|---|---|
| | bzw. $-N{<}^{R^1}_{R^2}$ | | |
| 12 | $CH(CH_3)_2$ | 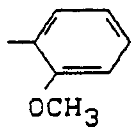 | Fp.: 72° C |
| 13 | $CH(CH_3)_2$ | 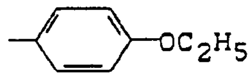 | Fp.: 34° C |
| 14 | $CH(CH_3)_2$ | 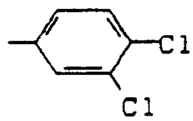 | Fp.: 64° C |
| 15 | $CH(CH_3)_2$ | 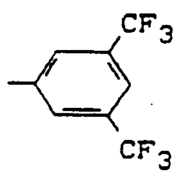 | Fp.: 103° C |

Tabelle 1 - Fortsetzung

| Beisp.-Nr. | $R^1$ | $R^2$<br>bzw. 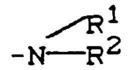 | Physikalische Daten |
|---|---|---|---|
| 16 | $CH(CH_3)_2$ | 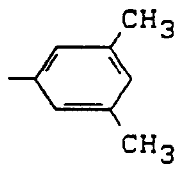 | Fp.: 95° C |
| 17 | $CH(CH_3)_2$ | 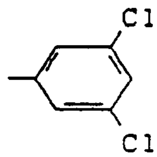 | Fp.: 111° C |
| 18 | $CH(CH_3)_2$ | 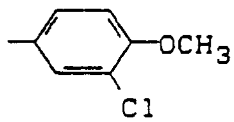 | Fp.: 150° C |
| 19 | $CH(CH_3)_2$ | 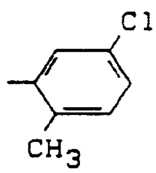 | Fp.: 88° C |

Man erhält die neuen Difluormethyl-thiadiazolyl-oxyessigsäureamide der allgemeinen Formel (I), wenn man 5-Difluormethyl-2-methylsulfonyl-1,3,4-thiadiazol der Formel (II)

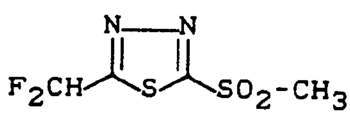 (II)

mit Hydroxyessigsäureamiden der allgemeinen Formel (III)

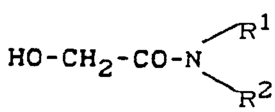 (III)

in welcher

$R^1$ und $R^2$ die oben angegebenen Bedeutungen haben,

gegebenenfalls in Gegenwart eines Verdünnungsmittels und gegebenenfalls in Gegenwart eines Säureakzeptors umsetzt.

Verwendet man 5-Difluormethyl-2-methylsulfonyl-1,3,4-thiadiazol und Hydroxyessigsäure-N-isopropylanilid als Ausgangsstoffe, so kann das Verfahren zur Herstellung der neuen Verbindungen der Formel (I) durch folgendes Formelschema skizziert werden:

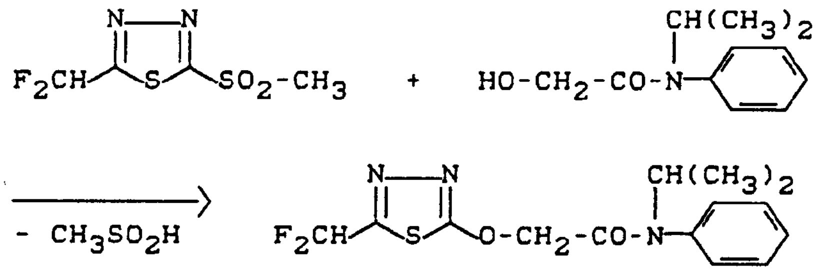


Das als Ausgangsstoff zu verwendende 5-Difluormethyl-2-methylsulfonyl-1,3,4-thiadiazol der formel (II) ist aus der Literatur bekannt.

Man erhält die Verbindung der Formel (II), wenn man 5-Difluormethyl-2-methylthio-1,3,4-thiadiazol der formel (IV)

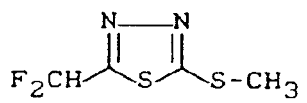
(IV)

mit einem Oxidationsmittel, wie z. B. Hydrogenperoxid, gegebenenfalls in Gegenwart eines Katalysators, wie z. B. Natriumwolframat, und gegebenenfalls in Gegenwart von Verdünnungsmitteln, wei z. B. Wasser, Ameisensäure und/oder Essigsäure, bei Temperaturen zwischen 0°C und 110°C umsetzt.

Das als Zwischenprodukt benötigte 5-Difluormethyl-2-methylthio-1,3,4-thiadiazol der Formel (IV) ist ebenfalls aus der Literatur bekannt.

Man erhält die Verbindung der Formel (IV), wenn man Difluoressigsäure mit Dithiocarbazinsäuremethylester in Gegenwart eines Säureakzeptors, wie z, B, Kaliumcarbonat, sowie gegebenenfalls in Gegenwart eines Verdünnungsmittels, wie z. B. Toluol, bei Temperaturen zwischen 0°C und 100°C umsetzt.

Die weiter als Ausgangsstoffe zu verwendenden Hydroxyessigsäureamide sind durch die Formel (III) allgemein definiert.

Als Beispiele für die Ausgangsstoffe der Formel (III) seien genannt:
N-Isopropyl-hydroxyessigsäure-anilid, -2-fluor-anilid, -3-fluor-anilid, -4-fluor-anilid, -3,4-difluor-anilid, -2,4-difluor-anilid, -2,6-difluor-anilid, -2-chlor-anilid, -3-chlor-anilid, -4-chlor-anilid, -2,4-dichlor-anilid, -3,4-dichloranilid, -2,6-dichlor-anilid, -2,5-dichlor-anilid, -3,5-dichlor-anilid, -2-chlor-6-fluor-anilid, -3-brom-anilid, -4-bromanilid, -3-cyano-anilid, -4-cyano-anilid, -2-methyl-anilid, -3-methyl-anilid, -4-methyl-anilid, -2,3-dimethyl-anilid, -2,4-dimethyl-anilid, -2,5-dimethyl-anilid, -3,4-dimethyl-anilid, -2-chlor-5-methyl-anilid, -5-chlor-2-methyl-anilid, -2-chlor-6-methyl-anilid, -3-ethyl-anilid, -4-ethyl-anilid, -3-trifluormethyl-anilid, -4-trifluormethyl-anilid, -2-trifluormethyl-anilid, -2-methoxy-anilid, -3-methoxy-anilid, -3-ethoxy-anilid und -4-ethoxy-anilid.

Die Hydroxyessigsäureamide der Formel (III) sind bekannt und/oder können nach an sich bekannten Verfahren hergestellt werden (vgl, EP-A 37 526, US-P 4 509 971, US-P 4 645 525, US 4 334 073).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z, B, Dimethylformamid, Dimethylacetamid und N-Methyl-pyrrolidon sowie Dimethylsulfoxid, Tetramethylensulfon, Hexamethylphosphorsäuretriamid und Wasser.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in monokotylen und dikotylen Kulturen, wie z. B. in Gerste, Weizen, Mais, Reis, Soja, Baumwolle, Rüben, Kartoffeln und Raps, insbesondere in Soja und Baumwolle, vor allem im Vorauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie

Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); 2-[(2-Chlorphenyl)-methyl]-4,4-dimethylisoxazolidin-3-on (DIMETHAZONE); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); N-Methyl-2-(1,3-benzthiazol-2-yl-oxy)-acetanilid (MEFENACET); 1-(3-Trifluormethyl-phenyl)-4-methylamino-5-chlor-6-pyridazon (NORFLURAZON); N-(1-Ethylpropyl)-3,4-dimethyl-2,6-dinitroanilin (PENDIMETHALIN); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0.05 und 5 kg pro ha.

Die Herstellung und die Verwendung der erfindungsgemäßen Wirkstoffe geht aus den nachfolgenden Beispielen hervor.

Verwendungsbeispiele

In den folgenden Verwendungsbeispielen wird die Verbindung der nachstehenden Formel als Vergleichssubstanz eingesetzt:

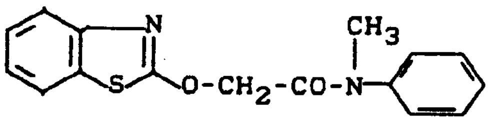 (A)

2-(Benzthiazol-2-yl-oxy)-essigsäure-N-methyl-anilid
(bekannt aus EP-A 5501 und US-P 4 509 971).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit gegenüber dem Stand der Technik zeigen in diesem Test z. B. die Verbindungen gemäß den Beispielen 1, 2, 3 und 4 aus Tabelle 1.

Herstellungsbeispiele

Beispiel 1

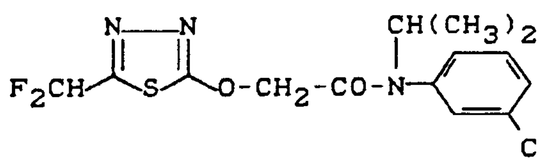

Eine Lösung von 1,44 g (0,036 Mol) Natriumhydroxid in 6 ml Wasser wird unter Rühren zu einer auf -20°C abgekühlten Mischung aus 6,4 g (0,03 Mol) 5-Difluormethyl-2-methylsulfonyl-1,3,4-thiadiazol, 7,2 g (0,03 Mol) Hydroxyessigsäure-N-isopropyl-(3-chlor-phenyl-amid) und 50 ml Aceton tropfenweise gegeben und die Reaktionsmischung wird 15 Stunden unter Kühlen mit einer Eis-Kochsalz-Mischung gerührt. Dann wird mit Essigsäure angesäuert und im Wasserstrahlvakuum eingeengt, Das beim anschließenden Verdünnen mit Wasser kristallin anfallende Produkt wird duch Absaugen isoliert.

Man erhält 11 g (~ 100 % der Theorie) 2-(5-Difluormethyl-1,3,4-thiadiazol-2-yl-oxy)-essigsäure-N-isopropyl(3-chlor-phenyl-amid) vom Schmelzpunkt 98°C.

Analog Beispiel 1 sowie entsprechend der allgemeinen Beschreibung des erfindungsgemäßen Herstellungsverfahrens können z, B, auch die weiteren in Tabelle 1 (oben) aufgeführten Verbindungen der Formel (I) hergestellt werden.

Ausgangsverbindung der Formel (II)

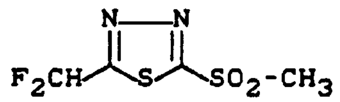

(II)

420 ml einer 35 %igen wäßrigen Hydrogenperoxidlösung werden unter Rühren zu einer auf 50 °C erwärmten Mischung aus 198 g (1,28 Mol) 5-Difluormethyl-2-methylthio-1,3,4-thiadiazol, 390 ml Ameisensäure und 0,8 g Natriumwolframat tropfenweise gegeben, wobei die Temperatur kurzzeitig auf 95 °C steigt. Dann wird das dabei kristallin angefallene Produkt durch Absaugen isoliert.

Man erhält 250 g (91 % der Theorie) 5-Difluormethyl-2-methylsulfonyl -1,3,4-thiadiazol vom Schemlzpunkt 59 °C.

Ausgangsverbindung der Formel (IV)

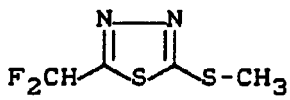

(IV)

131 g (1,29 Mol) Difluoressigsäure werden bei 20 °C unter Rühren zu einer Mischung aus 89 g (0,65 Mol) Kaliumcarbonat und 130 ml Wasser tropfenweise gegeben. Nach Zugabe von 300 ml Toluol wird das Wasser durch azeotrope Destillation am Wasserabscheider entfernt. Zur verbleibenden organischen Phase werden weitere 700 ml Toluol und 156 g (1,29 Mol) Dithiocarbazinsäure-methylester gegeben. Dann werden bei 50 °C bis 60 °C 404 g (1,61 Mol) Phosphoroxychlorid zugetropft, die Mischung wird 2 Stunden gerührt und anschließend auf Eiswasser gegossen. Die organische Phase wird abgetrennt, mit Wasser gewaschen, mit Natriumsulfat getrocknet und filtriert. Vom Filtrat wird das Lösungsmittel im Wasserstrahlvakuum abdestilliert.

Man erhält 190 g (96 % der Theorie) 5-Difluormethyl-2-methylthio-1,3,4-thiadiazol als amorphen Rückstand, welcher allmählich kristallisiert. Schmelzpunkt: 23 °C.

**Patentansprüche**

1. Difluormethyl-thiadiazolyl-oxyessigsäureamide der allgemeinen Formel (I)

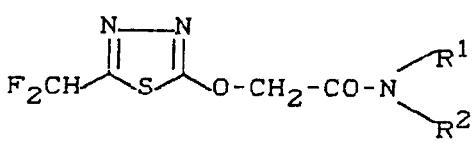

(I)

in welcher

$R^1$ für Isopropyl steht und

$R^2$ für gegebenenfalls durch Fluor, Chlor, Brom, Methyl, Ethyl, Trifluormethyl, Methoxy und/oder Ethoxy substituiertes Phenyl steht.

2. Verfahren zur Herstellung von Difluormethyl-thiadiazolyl-oxyessigsäureamiden der Formel (I) gemäß Anspruch 1, dadurch gekennzeichnet, daß man 5-Difluormethyl-2-methylsulfonyl-1,3,4-thiadiazol der Formel (II)

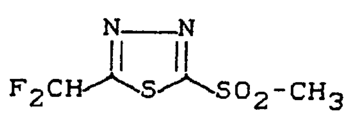

(II)

mit Hydroxyessigsäureamiden der allgemeinen Formel (III)

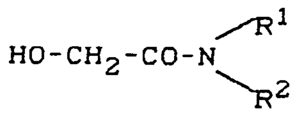

(III)

in welcher

$R^1$ und $R^2$ die in Anspruch 1 angegebenen Bedeutungen haben,

gegebenenfalls in Gegenwart eines Verdünnungsmittels und gegebenenfalls in Gegenwart eines Säureakzeptors umsetzt.

3. Selektiv-herbizide Mittel, gekennzeichnet durch einen Gehalt an mindestens einem Difluormethyl-thiadiazolyl-oxyessigsäureamid der Formel (I) gemäß Anspruch 1.

4. Verfahren zur selektiven Bekämpfung von Unkraut in Nutzpflanzenkulturen, dadurch gekennzeichnet, daß man Difluormethyl-thiadiazolyl-oxyessigsäureamide der Formel (I) gemäß Anspruch 1 auf Unkraut und/ oder seinen Lebensraum einwirken läßt.

5. Verwendung von Difluormethyl-thiadiazolyl-oxyessigsäureamiden der Formel (I) gemäß Anspruch 1 zur selektiven Bekämpfung von Unkraut in Nutzpflanzenkulturen.

6. Verfahren zur Herstellung von selektiv-herbiziden Mitteln, dadurch gekennzeichnet, daß man Difluormethyl-thiadiazolyl-oxyessigsäureamide der Formel (I) gemäß Anspruch 1 mit Streckmitteln und/oder oberflächenaktiven Mitteln vermischt.

## Claims

1. Difluoromethyl-thiadiazolyl-oxyacetamides of the general formula (I)

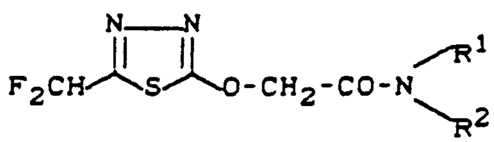

(I)

in which

$R^1$ represents isopropyl and

$R^2$ represents phenyl which is optionally substituted by fluorine, chlorine, bromine, methyl, ethyl, trifluoromethyl, methoxy and/or ethoxy.

2. Process for the preparation of difluoromethyl-thiadiazolyl-oxyacetamides of the formula (I) according to Claim 1, characterised in that 5-difluoromethyl-2-methylsulphonyl-1,3,4-thiadiazole of the formula (II)

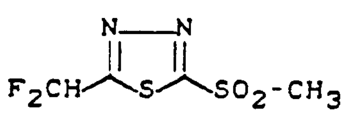

(II)

is reacted with hydroxyacetamides of the general formula (III)

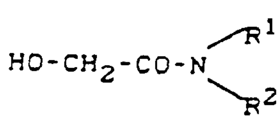

(III)

in which

$R^1$ and $R^2$ have the meanings specified in Claim 1,

if appropriate in the presence of a diluent and if appropriate in the presence of an acid acceptor.

3. Selectively-herbicidal agents, characterised in that they contain at least one difluoromethyl-thiadiazolyl-oxyacetamide of the formula (I) according to Claim 1.

4. Method of selectively combating weeds in crops, characterised in that difluoromethyl-thiadiazolyl-oxyacetamides of the formula (I) according to Claim 1 are allowed to act on the weeds and/or their environment.

5. Use of difluoromethyl-thiadiazolyl-oxyacetamides of the formula (I) according to Claim 1 for selectively combating weeds in crops.

6. Process for preparing selectively-herbicidal agents, characterised in that difluoromethyl-thiadiazolyl-oxyacetamides of the formula (I) according to Claim 1 are mixed with extenders and/or surface-active agents.

## Revendications

1. Difluorométhyl-thiadiazolyl-oxyacétamides de formule générale I

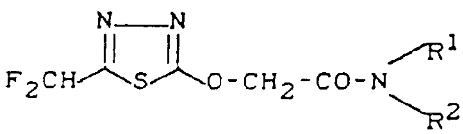

(I)

dans laquelle

$R^1$ représente un groupe isopropyle et

$R^2$ représente un groupe phényle éventuellement substitué par le fluor, le chlore, le brome, des groupes méthyle, éthyle, trifluorométhyle, méthoxy et/ou éthoxy.

2. Procédé de préparation des difluorométhyl-thiadiazolyl-oxyacétamides de formule I de la revendication 1, caractérisé en ce que l'on fait réagir le 5-difluorométhyl-2-méthylsulfonyl-1,3,4-thiadiazole de formule II

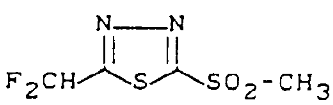

(II)

avec des hydroxyacétamides de formule générale III,

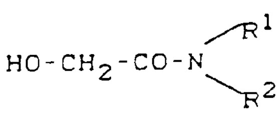

(III)

dans laquelle

$R^1$ et $R^2$ ont les significations indiquées dans la revendication 1, éventuellement en présence d'un diluant et éventuellement en présence d'un accepteur d'acide.

3. Produit herbicide sélectif, caractérisé en ce qu'il contient au moins un difluorométhyl-thiadiazolyl-oxyacétamide de formule I de la revendication 1.

4. Procédé pour combattre sélectivement les végétaux adventices dans les cultures de végétaux utiles, caractérisé en ce que l'on applique sur les végétaux adventices et/ou leur habitat des difluorométhyl-thiadiazolyl-oxyacétamides de formule I de la revendication 1.

5. Utilisation des difluorométhyl-thiadiazolyl-oxyacétamides de formule I de la revendication 1 pour la lutte sélective contre les végétaux adventices dans les cultures de végétaux utiles.

6. Procédé de préparation de produits herbicides sélectifs, caractérisé en ce que l'on mélange des difluorométhyl-thiadiazolyl-oxyacétamides de formule I de la revendication 1 avec des diluants et/ou des agents tensioactifs.